(19) Europäisches Patentamt / European Patent Office / Office européen des brevets

(11) **EP 4 414 993 B1**

(12) **EUROPEAN PATENT SPECIFICATION**

(45) Date of publication and mention
of the grant of the patent:
**10.06.2026   Bulletin 2026/24**

(51) International Patent Classification (IPC):
**G16C 60/00** *(2019.01)*      **G06N 20/00** *(2019.01)*
G16C 20/30 *(2019.01)*      G16C 20/70 *(2019.01)*

(21) Application number: **22878389.0**

(22) Date of filing: **28.09.2022**

(52) Cooperative Patent Classification (CPC):
**G16C 60/00; G06N 20/00;** G16C 20/30;
G16C 20/70; Y02P 90/30

(86) International application number:
**PCT/JP2022/036163**

(87) International publication number:
**WO 2023/058519 (13.04.2023 Gazette 2023/15)**

(54) **COMPOSITION SEARCH METHOD**

ZUSAMMENSETZUNGSSUCHVERFAHREN

PROCÉDÉ DE RECHERCHE DE COMPOSITION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.10.2021   JP 2021163338**

(43) Date of publication of application:
**14.08.2024   Bulletin 2024/33**

(73) Proprietor: **Resonac Corporation
Tokyo 105-7325 (JP)**

(72) Inventors:
 • **LEE, Haein
 Tokyo 105-8518 (JP)**
 • **MINAMI, Takuya
 Tokyo 105-8518 (JP)**
 • **OKUNO, Yoshishige
 Tokyo 105-8518 (JP)**

(74) Representative: **Strehl & Partner mbB
 Maximilianstrasse 54
 80538 München (DE)**

(56) References cited:
**WO-A1-2021/045058      JP-A- 2020 128 962
JP-A- 2020 187 417**

• **IKEDA YUKO ET AL: "Materials Informatics Approach to Predictive Models for Elastic Modulus of Polypropylene Composites Reinforced by Fillers and Additives", vol. 7, 11 June 2021 (2021-06-11), pages 1 - 8, XP093056379, Retrieved from the Internet <URL:https://www.jstage.jst.go.jp/article/jccjie/7/0/7_2020-0007/_pdf/-char/en> DOI: 10.2477/jccjie.2020-0007**
• **VENKATRAMAN VISHWESH ED - MISHNAEVSKYJR LEON ET AL: "The utility of composition-based machine learning models for band gap prediction", COMPUTATIONAL MATERIALS SCIENCE, ELSEVIER, AMSTERDAM, NL, vol. 197, 17 June 2021 (2021-06-17), XP086707514, ISSN: 0927-0256, [retrieved on 20210617], DOI: 10.1016/J.COMMATSCI.2021.110637**
• **IKEDA YUKO, OKUYAMA MICHIHIRO, NAKAZAWA YUKIHITO, OSHIYAMA TOMOHIRO, FUNATSU KIMITO: "Materials Informatics Approach to Predictive Models for Elastic Modulus of Polypropylene Composites Reinforced by Fillers and Additives", JOURNAL OF COMPUTER CHEMISTRY, JAPAN -INTERNATIONAL EDITION, vol. 7, 11 June 2021 (2021-06-11), pages 1 - 8, XP093056379, DOI: 10.2477/jccjie.2020-0007**

## Description

TECHNICAL FIELD

The present invention relates to a composition search method.

BACKGROUND

In material design, it is necessary to determine parameters (a composition or a composition ratio, and a constraint condition such as cost and a manufacturing condition) for obtaining a value of a physical property of a target material. Conventionally, an experimenter often determines parameters empirically or by trial and error. However, in a case of a complicated material design with a large number of parameters, it takes a long time and is extremely difficult to obtain a target physical property.

In order to improve such a conventional material design, a technique of obtaining an optimum parameter by performing machine learning using accumulated data in which the above-described parameters are associated with known physical properties is proposed in recent years.

As an example, Patent Document 1 below proposes an optimization method of generating a Bayesian model for searching for a combination of values of multiple parameters that gives an optimum value as a value of a physical property related to a target substance, and performing a search for the combination using the Bayesian model in a search space.

Additionally, Non-Patent Document 1 below proposes a technique that is one of sequential search methods using a prediction model, that determines a next candidate point by using a distance between a prediction value and a training data value, and that optimizes a hyperparameter of the model. According to this method, the prediction method is not limited in the parameter search.

As another example, Patent Document 2 below proposes searching for a design condition so as to reduce variations in multiple predicted values that are obtained based on multiple different training datasets, when searching for a parameter in which a desired physical property can be obtained, by using a prediction model that predicts a value of a physical property from a design parameter of a metallic material, searching for a parameter including a new region different from past actual data so as to increase a difference between the parameter and a parameter in the past actual data. IKEDA YUKO ET AL: "Materials Informatics Approach to Predictive Models for Elastic Modulus of Polypropylene Composites Reinforced by Fillers and Additives", JOURNAL OF COMPUTER CHEMISTRY, JAPAN - INTERNATIONAL EDITION, vol. 7, 11 June 2021, pages 1-8, discloses model-based composition screening and distance to training data.

Related Art Document

Patent Document

Patent Document 1: Japanese Laid-open Patent Application Publication No. 2020-187642
Patent Document 2: International Publication No. WO 2020-152993

[Non-Patent Document]

Non-Patent Document 1: DOI: arxiv-2101.02289

SUMMARY OF THE INVENTION

Problem to be solved by the invention

However, the invention disclosed in Patent Document 1 uses a Bayesian model, and is limited to an optimization method of Gaussian process regression. Therefore, there is a problem that another prediction method (for example, gradient boosting, a neural network, or the like) expected to have high prediction performance cannot be flexibly used, and the prediction method is limited.

In the technique described in Non-Patent Document 1, the prediction method is not limited in the parameter search. In the case of the technique described in Non-Patent Document 1, prediction accuracy verified with past parameters is weighted on a term of a distance from training data so that a parameter away from the past parameters can be searched for in consideration of the accuracy of the prediction model. However, in the case of the technique described in Non-Patent Document 1, the weighting is uniformly performed on all parameters, and a search is uniformly performed including a parameter having a small relationship with an objective variable. Therefore, there is a problem that it takes time to reach the optimum parameter.

Additionally, the invention disclosed in Patent Document 2 is configured to apply a weight to each parameter so that a difference from a parameter in past actual data increases, but the weight is determined by a user, which is arbitrary, and therefore, there is a problem that the search is not necessarily performed appropriately.

It is an object of the present invention to provide a composition search method of searching for a composition for obtaining a target value of a physical property more efficiently.

Means for Solving the Problem

The present invention is defined in the appended claims.

The present invention is directed to a composition search method for a material including:

a step of constructing a prediction model by learning training data in which information related to a composition of a material is set as an explanatory variable and a value of a physical property of the material is set as an objective variable;

a step of calculating a predicted value of the physical property by inputting, into the prediction model, prediction data for newly searching for a composition;

a step of calculating an influence degree of each explanatory variable on prediction by using the training data and the prediction model;

a step of calculating a weighted distance of the prediction data with respect to the training data by using the influence degree; and

a step of displaying a relationship between the predicted value and the weighted distance, and outputting corresponding prediction data as a search candidate.

Effect of the invention

According to the present disclosure, a composition for obtaining a target value of a physical property can be searched for more efficiently.

BRIEF DESCRIPTION OF THE DRAWINGS

[FIG. 1] FIG. 1 is a first diagram illustrating an example of a system configuration of a composition search system.
[FIG. 2] FIG. 2 is a diagram illustrating an example of a hardware configuration of a learning device and a predicting device.
[FIG. 3] FIG. 3 is a diagram illustrating an example of training data and prediction data.
[FIG. 4] FIG. 4 is a first diagram illustrating an example of a graph indicating a relationship between a predicted value and a weighted distance.
[FIG. 5] FIG. 5 is a first flowchart illustrating a flow of a composition search process.
[FIG. 6] FIG. 6 is a second diagram illustrating an example of the system configuration of the composition search system.
[FIG. 7] FIG. 7 is a second diagram illustrating an example of the graph indicating the relationship between the predicted value and the weighted distance.
[FIG. 8] FIG. 8 is a third diagram illustrating an example of the graph indicating the relationship between the predicted value and the weighted distance.
[FIG. 9] FIG. 9 is a second flowchart illustrating a flow of the composition search process.
[FIG. 10] FIG. 10 is a third diagram illustrating an example of the system configuration of the composition search system.
[FIG. 11] FIG. 11 is a third flowchart illustrating a flow of the composition search process.
[FIG. 12] FIG. 12 is a graph indicating the number of times for ending of search in an example and comparative examples.

DESCRIPTION OF THE EMBODIMENTS

In the following, each embodiment will be described with reference to the accompanying drawings. In order to facilitate understanding of the description, the same reference symbols are given to the same components in the drawings as far as possible, and duplicated description will be omitted.

[First Embodiment]

A composition search method according to a first embodiment includes: a step of constructing a prediction model by learning training data in which information related to a composition of a material is set as an explanatory variable and a value of a physical property of the material is set as an objective variable; a step of calculating a predicted value of the physical property by inputting, into the prediction model, prediction data for newly searching for a composition; a step of calculating an influence degree of each explanatory variable on prediction by using the training data and the prediction model; a step of calculating a weighted distance of the prediction data with respect to the training data by using the influence degree; and a step of displaying a relationship between the predicted value and the weighted distance and outputting corresponding prediction data as a search candidate.

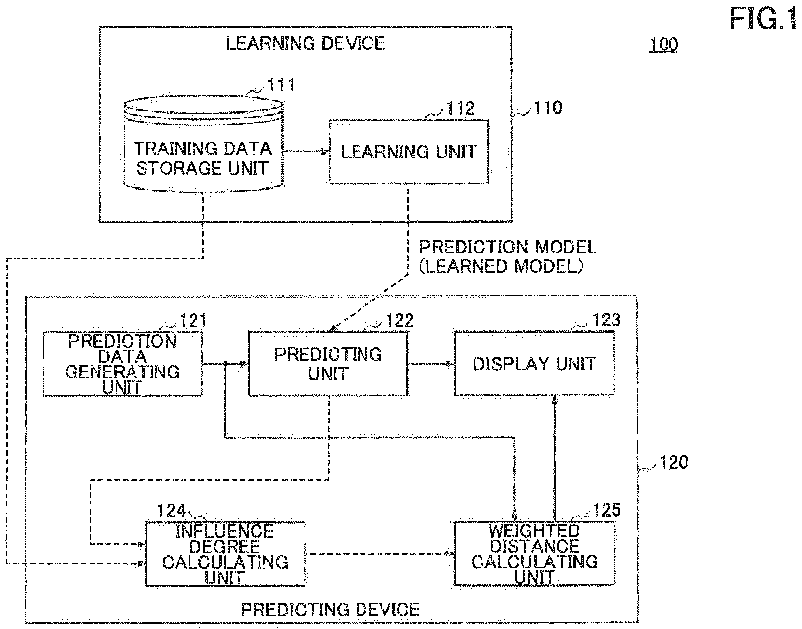

Here, in the present specification, the composition may be elements constituting an alloy material, or may be various raw materials constituting an organic material or a composite material. Additionally, in the present specification, a type, a preparation ratio, a feature, and the like of the raw material, which are information related to the composition, are also referred to as parameters of the raw material. Hereinafter, the details of the composition search method according to the first embodiment will be described using FIG. 1 to FIG. 5.

<System Configuration of Composition Search System>

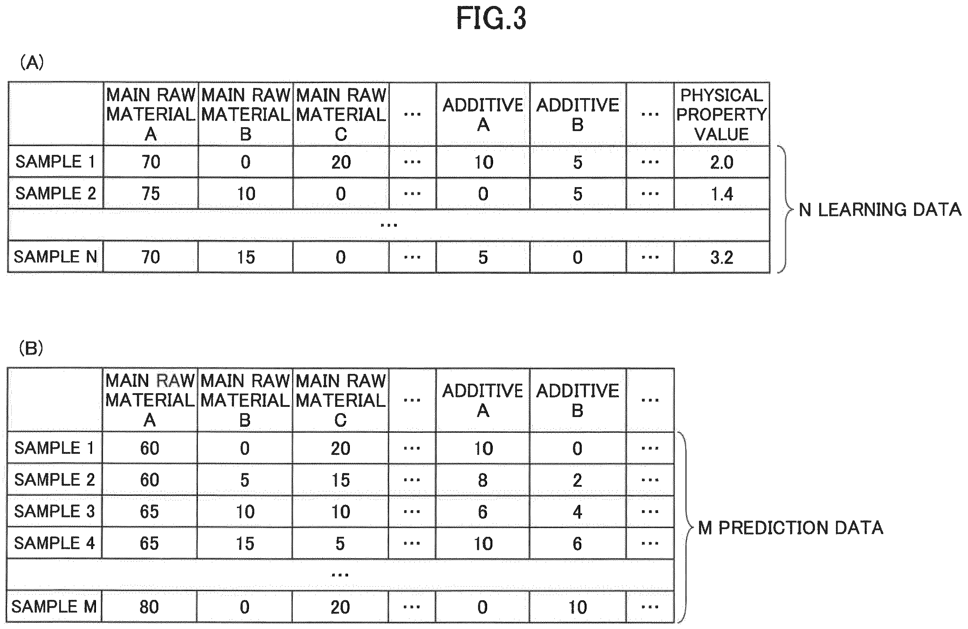

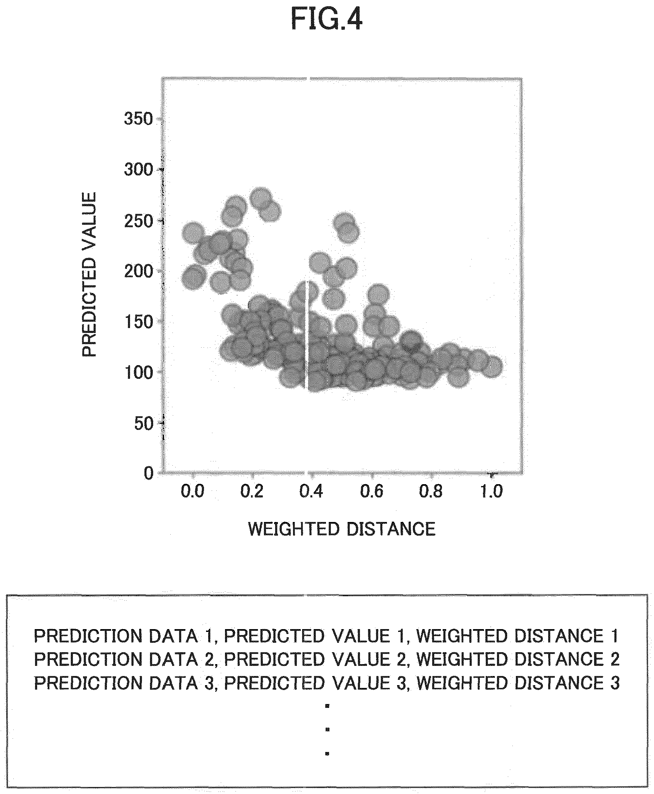

First, a system configuration of a composition search system for realizing the composition search method according to the first embodiment will be described using FIG. 1 with reference to FIG. 3 and FIG. 4. FIG. 1 is a first diagram illustrating an example of the system configuration of the composition search system. FIG. 3 is a diagram illustrating an example of the training data and the prediction data. FIG. 4 is a first diagram illustrating an example of a graph indicating the relationship between the predicted value and the weighted distance.

As illustrated in FIG. 1, a composition search system 100 includes a learning device 110 and a predicting device 120.

A learning program is installed in the learning device 110, and the learning device 110 functions as a learning unit 112 by executing the program.

The learning unit 112 constructs a prediction model (a learned model) by using the training data stored in a training data storage unit 111. In the present embodiment, the training data used when the learning unit 112 constructs the prediction model includes a set of the parameters of the raw material (the type, the preparation ratio, the feature) and a measured value of the physical property for multiple experimental samples (see FIG. 3 (A)).

Additionally, in the present embodiment, the model trained by the learning unit 112 includes any method such as random forest, Gaussian process regression, a neural network, and an ensemble learning model combining multiple methods.

Here, the prediction model (the learned model) constructed by the learning unit 112 is set in a predicting unit 122 of the predicting device 120.

A predicting program is installed in the predicting device 120, and the predicting device 120 functions as a prediction data generating unit 121, the predicting unit 122, a display unit 123, an influence degree calculating unit 124, and a weighted distance calculating unit 125 by executing the program.

The prediction data generating unit 121 generates the prediction data. The prediction data includes data of combinations of compositions exhaustively generated according to a constraint condition defining upper and lower limits and a step size of a composition ratio, raw materials that cannot be used at the same time, and the like, or features related to the compositions (see FIG. 3 (B)). Here, the prediction data generating unit 121 inputs the generated prediction data into the predicting unit 122 and notifies the weighted distance calculating unit 125 of the prediction data.

The predicting unit 122 calculates a predicted value from the prediction data by using the prediction model. Additionally, the predicting unit 122 notifies the display unit 123 of the calculated predicted value.

The influence degree calculating unit 124 calculates the influence degree of each explanatory variable on the prediction using the training data stored in the training data storage unit 111 and the prediction model. Specifically, the influence degree calculating unit 124 calculates the influence degree by using various algorithms stored in various Python libraries.

For example, when the prediction model is a linear model, the influence degree calculating unit 124 calculates the influence degree by using a coefficient of each variable. Additionally, when the prediction model is a model based on a decision tree, the influence degree calculating unit 124 calculates the influence degree, such as permutation importance or Gini importance. Alternatively, the influence degree calculating unit 124 may calculate the influence degree by using an algorithm of SAGE or SHAP of a Python library, which can calculate the influence degree in a selected method.

The weighted distance calculating unit 125 calculates the weighted distance of the prediction data with respect to the training data by using the influence degree calculated by the influence degree calculating unit 124. Specifically, the weighted distance calculating unit 125 calculates the weighted distance by using the following Equations (2) and (3).

[Eq. 2]

$$d_n = \sqrt{\sum_{t=1}^{k} w_t \times (X_{n_t} - x_{n_t})^2} \qquad (2)$$

[Eq. 3]

$$D_t = \frac{1}{N} \sum_{n=1}^{N} d_n \qquad (3)$$

Here, $d_n$ is a weighted average distance between the n-th prediction data and the training data, N is the total number of the experiments in which measurements are performed, k is the total number of the explanatory variables (the parameters of the raw material), $X_{nt}$ is the t-th explanatory variable in the n-th training data, $x_{nt}$ is the t-th explanatory variable in the n-th prediction data, and $w_t$ is the influence degree. The weighted distance $D_i$ is a value obtained by scaling the calculated $d_n$ to a value between zero and one, inclusive.

The display unit 123 displays multiple relationships between the prediction values calculated by the predicting unit 122 and the weighted distances calculated by the weighted distance calculating unit 125. For example, the display unit 123 displays multiple relationships between the predicted values and the weighted distances by using a two dimensional graph in which the horizontal axis represents the weighted distance and the vertical axis represents the predicted value (see FIG. 4). Additionally, the display unit 123 outputs the corresponding prediction data as the search candidate.

<Hardware Configuration of Learning Device and Predicting Device>

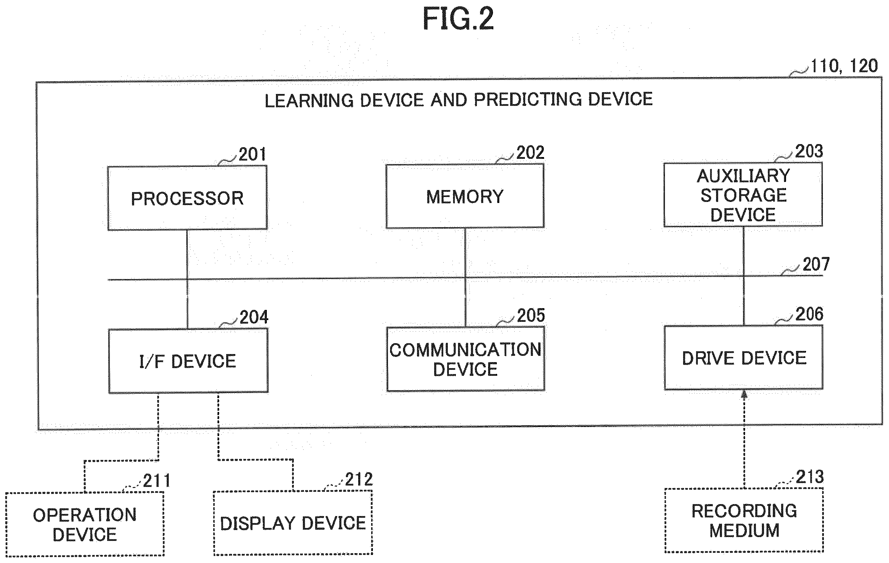

Next, a hardware configuration of the learning device 110 and the predicting device 120 included in the composition search system 100 will be described. Here, in the present embodiment, the hardware configuration of the learning device 110 and the hardware configuration of the predicting device 120 are substantially the same, and therefore, here, the configurations will be described together with reference to FIG. 2. FIG. 2 is a diagram illustrating an example of the hardware configuration of the learning device and the predicting device.

As illustrated in FIG. 2, the learning device 110 and the predicting device 120 include a processor 201, a memory 202, an auxiliary storage device 203, an interface (I/F) device 204, a communication device 205, and a drive device 206. Here, hardware components of each of the learning device 110 and the predicting device 120 are connected to each other via a bus 207.

The processor 201 includes various arithmetic devices, such as a central processing unit (CPU), a graphics processing unit (GPU), and the like. The processor 201 reads various programs (for example, a learning program, a predicting program, and the like) into the memory 202 and executes the programs.

The memory 202 includes a main storage device, such as a read only memory (ROM) or a random access memory (RAM). The processor 201 and the memory 202 form what is called a computer, and by the processor 201 executing various programs read into the memory 202, the computer realizes various functions.

The auxiliary storage device 203 stores various programs and various data used when the various programs are executed by the processor 201. For example, the training data storage unit 111 is realized in the auxiliary storage device 203.

The I/F device 204 is a connection device that connects to an operation device 211 and a display device 212, which are examples of user interface devices. The communication device 205 is a communication device for communicating with an external device (not illustrated) via a network.

The drive device 206 is a device in which a recording medium 213 is set. The recording medium 213 herein includes a medium for optically, electrically, or magnetically recording information, such as a CD-ROM, a flexible disk, or a magnetooptical disk. Additionally, the recording medium 213 may include a semiconductor memory or the like that electrically records information, such as a ROM or a flash memory.

Here, the various programs to be installed in the auxiliary storage device 203 are installed by, for example, the distributed recording medium 213 being set in the drive device 206 and the various programs recorded in the recording medium 213 being read by the drive device 206. Alternatively, the various programs to be installed in the auxiliary storage device 203 may be installed by being downloaded from the network via the communication device 205.

<Flow of Composition Search Process in Composition Search System>

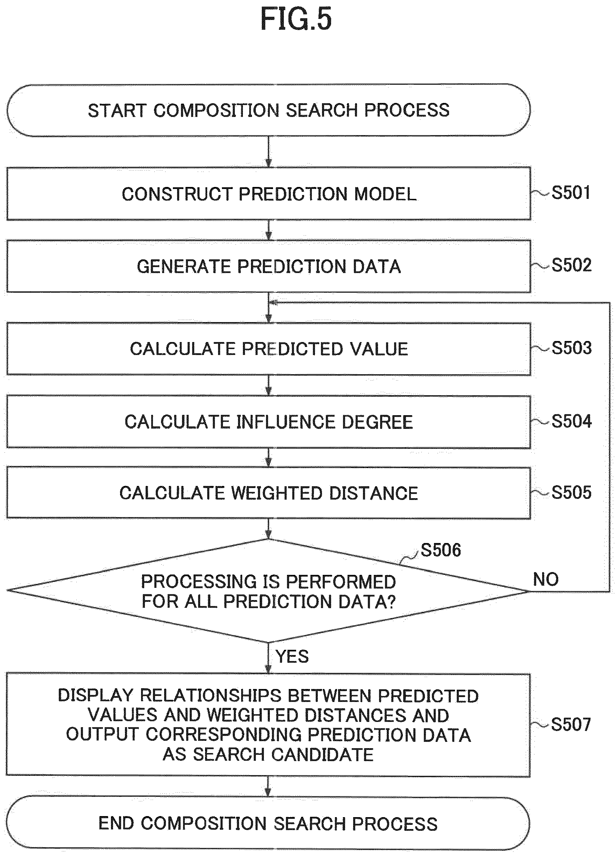

Next, a flow of a composition search process in the composition search system 100 will be described. FIG. 5 is a first flowchart illustrating the flow of the composition search process.

In step S501, the learning device 110 constructs the prediction model. As described above, in the present embodiment, the training data used when the learning device 110 constructs the prediction model includes a set of the parameters (the type, the preparation ratio, and the feature) of the raw material and the measured value of the physical property for multiple experimental samples (see FIG. 3 (A)).

Additionally, as described above, the prediction model constructed by the learning device 110 is a learned model obtained by performing machine learning using the training data in which the parameter of the raw material of the training data is the explanatory variable and the measured value of the physical property is the objective variable.

In step S502, the predicting device 120 generates the prediction data. As described above, the prediction data generated by the predicting device 120 in the present embodiment includes data of combinations of compositions exhaustively generated according to the constraint condition defining the upper and lower limits and the step size of the composition ratio, the raw materials that cannot be used at the same time, and the like or the features related to the compositions (see FIG. 3 (B)).

In step S503, the predicting device 120 calculates the predicted value from the prediction data by using the prediction model constructed in step S501.

In step S504, the predicting device 120 calculates the influence degree of each explanatory variable on the prediction by using the training data and the prediction model.

In step S505, the predicting device 120 calculates the weighted distance of the prediction data to the training data by using the influence degrees calculated in step S504.

In step S506, the predicting device 120 checks whether the predicted value and the weighted distance have been calculated for all the prediction data. If the predicted value and the weighted distance have been calculated for all the prediction data (YES in step S506), the process proceeds to step S507. If there is prediction data for which the predicted value and the weighted distance have not been calculated (NO in step S506), the process returns to step S503.

In step S507, the predicting device 120 displays multiple relationships between the predicted values and the weighted distances, and outputs the corresponding prediction data as the search candidate. As described above, when displaying multiple relationships between the predicted values and the weighted distances, the predicting device 120 plots and displays the predicted values on a two dimensional graph in which the horizontal axis represents the weighted distance and the vertical axis represents the predicted value (see FIG. 4).

<Effects of Composition Search Method According to First Embodiment>

Next, the effects of the composition search method according to the first embodiment will be described. In the case of the composition search method according to the first embodiment, the user can select the search candidate in consideration of the predicted value and the weighted distance of the prediction data with respect to the training data.

To begin with, in the prediction of the value of the physical property, if a difference in an important parameter among information related to the composition is great, the actual values of the physical property are highly likely to greatly differ. With respect to the above, if the difference in the important parameter is great, the reliability of the predicted value predicted by the predicting device 120 is reduced.

Here, the unweighted distance is not suitable to be used as an index of the reliability of the predicted value because the important parameter is buried in the information related to the composition and is uniformly handled. That is, the weighted distance used in the first embodiment is more appropriate as an index indicating whether the reliability of the predicted value is higher or more challenging than the unweighted distance. As a result, according to the first embodiment, for example, by selecting a composition with a long weighted distance, the user can obtain a challenging search candidate for which focused searching in the important parameter is performed.

As described above, according to the first embodiment, because the search candidate can be selected while balancing the level of the reliability and the level of the challenge property of the predicted value, the composition for obtaining the target physical property value can be more efficiently searched for.

[Second Embodiment]

Next, a composition search method according to a second embodiment will be described focusing on differences from the first embodiment.

<System Configuration of Composition Search System>

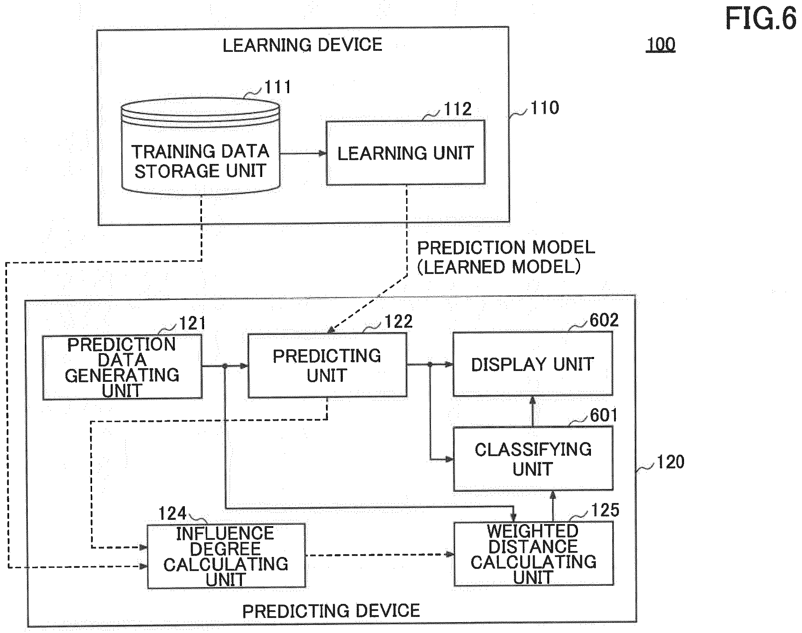

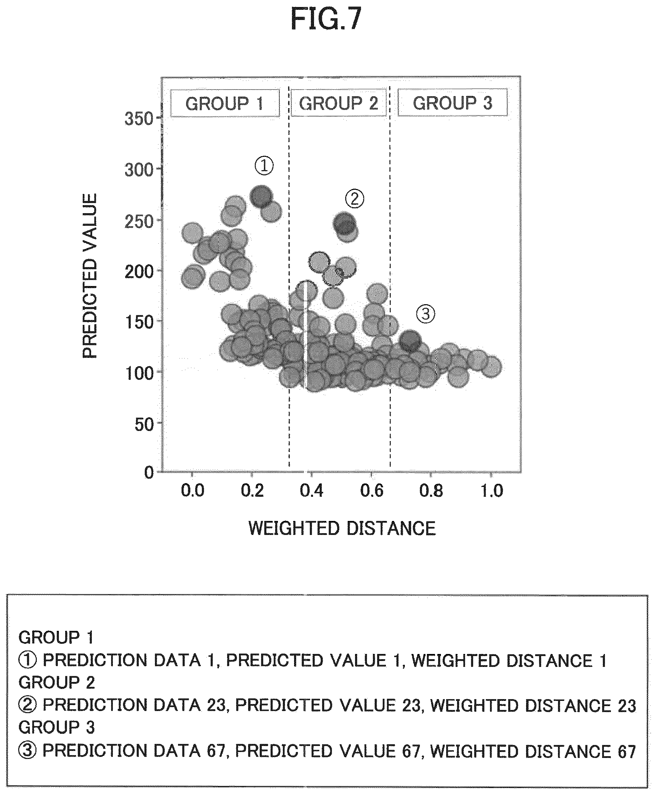

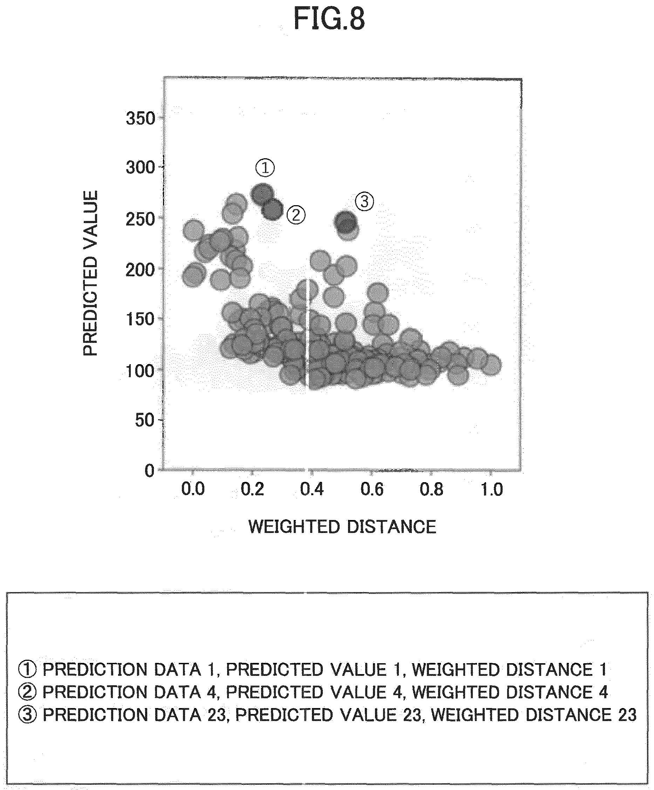

First, a system configuration of a composition search system that realizes the composition search method according to the second embodiment will be described using FIG. 6 with reference to FIG. 7 and FIG. 8. FIG. 6 is a second diagram illustrating an example of the system configuration of the composition search system. FIG. 7 and FIG. 8 are second and third diagrams illustrating examples of the graph indicating the relationship between the predicted value and the weighted distance.

The differences from the system configuration described with reference to FIG. 1 in the first embodiment are that, in the case of the system configuration illustrated in FIG. 6, the predicting device 120 includes a classifying unit 601, and a function of a display unit 602 is different from the function of the display unit 123.

The classifying unit 601 groups the predicted values calculated by the predicting unit 122 based on the weighted distance of the prediction data with respect to the training data. Additionally, the classifying unit 601 notifies the display unit 602 of a result of the grouping. Here, the grouping method by the classifying unit 601 may be selected suitably, and for example, either a method of equally dividing the weighted distance by a predetermined value between zero and one or a method of dividing the weighted distance such that the number of data in each group after the dividing is identical may be selected. Additionally, the number of groups may be a number set in advance or a number set by the user.

Additionally, the classifying unit 601 calculates an acquisition function serving as a reference when determining whether the prediction data is the search candidate, and notifies the display unit 602 of a result of the calculating. Specifically, the classifying unit 601 calculates the acquisition function using the following Equation (4), for example.
[Eq. 4]

$$Acq(X_i) = (1 - s_g) * f(X_i) + s_g * D_i \qquad (0 \le s_g \le 1) \qquad (4)$$

Here, $X_i$ is the i-th prediction data, $Acq(X_i)$ is the acquisition function of the i-th prediction data, $f(X_i)$ is a value obtained by scaling the predicted value of the i-th prediction data to a value between zero and one, inclusive, $s_g$ is a weighting factor in the g-th group, and $D_i$ is the weighted distance of the i-th prediction data to the training data. $s_g$ may be set to 0 in all the groups. In that case, the acquisition function $Acq(X_i)$ is equal to the predicted value $f(X_i)$. $s_g$ can be set by the user, and when $s_g$ is not 0 in all the groups, the candidate selection can be achieved in which consideration is given to the weighted distance ($D_i$) with respect to the training data in the group.

The display unit 602 displays multiple relations between the predicted values and the weighted distances, and outputs the corresponding prediction data as the search candidate in the order in which the acquisition function is higher for each group. Specifically, the prediction data (the information related to the composition) is selected from each group in the order in which the acquisition function is higher, and is output as the search candidate.

Here, the number of the search candidates output from each group can be appropriately set for each group, and can be set by the user in consideration of an experimental environment. For example, the user may set it such that the search candidates are equally output in each group. Alternatively, the user may set it such that the number of the search candidates output from a group having a long weighted distance is greater. In this case, the search can be performed with an emphasis on a composition having a long weighted distance with respect to the training data.

The example of FIG. 7 indicates a state in which, when multiple relationships between the predicted values and the weighted distances are displayed, the predicted values are plotted on a two dimensional graph with the weighted distances on the horizontal axis and the predicted values on the vertical axis, and the predicted values for which the acquisition function is high are displayed with numbering, and the corresponding prediction data is output as the search candidate.

Here, the above description assumes that the classifying unit 601 groups the prediction values and calculates the acquisition function, and the display unit 602 displays the predicted value for which the acquisition function is high with numbering for each group and outputs the corresponding prediction data as the search candidate.

However, the functions of the classifying unit 601 and the display unit 602 are not limited to this, and for example, the classifying unit 601 may be configured to calculate the acquisition function without grouping the predicted values, and the display unit 602 may be configured to display, with numbering, the predicted values for which the acquisition function is high and output the corresponding prediction data as the search candidate.

In this case, the classifying unit 601 may select the prediction data based on an acquisition function calculated using, for example, the following Equation (5) or Equation (6), and output the prediction data as the search candidate.
[Eq. 5]

$$Acq(X_i) = (1 - \alpha) * f(X_i) + \alpha * D_i \qquad (0 \le \alpha \le 1) \qquad (5)$$

[Eq. 6]

$$Acq(X_i) = (1 - \alpha) * f(X_i) + \alpha * (1 - D_i) \qquad (0 \le \alpha \le 1) \qquad (6)$$

Here, $X_i$ is the i-th prediction data, $Acq(X_i)$ is the acquisition function of the i-th prediction data, $f(X_i)$ is a value obtained by scaling the predicted value of the i-th prediction data to a value between zero and one, inclusive, $D_i$ is the weighted distance of the i-th prediction data with respect to the training data, and $\alpha$ is the weighting factor in $D_i$.

According to the classifying unit 601, the user can adjust which of the predicted value $f(X_i)$ and the weighted distance $D_i$ or $1-D_i$ is to be emphasized by appropriately setting the weighting factor $\alpha$ included in the acquisition function. For example, in the case of Equation (5), when $\alpha$ is increased, a high predicted value $f(X_i)$ can be searched for, while putting an emphasis on a composition having a long weighted distance with respect to the training data. Conversely, in the case of Equation (6), when $\alpha$ is decreased, a high predicted value $f(X_i)$ can be searched for, while putting an emphasis on a composition having a close weighted distance with respect to the training data and a high reliability of the predicted value.

Additionally, in the case of the classifying unit 601 described above, the display unit 602 selects the prediction data in the order in which the acquired function is higher and outputs the prediction data as the search candidate (see FIG. 8). Here, the display unit 602 can use either Equation (5) or Equation (6), or both as the acquisition function. When both of the equations are used, the number of the search candidates to be output by each of the equations may be appropriately set in consideration of the total number of the search candidates to be output.

<Flow of Composition Search Process in Composition Search System>

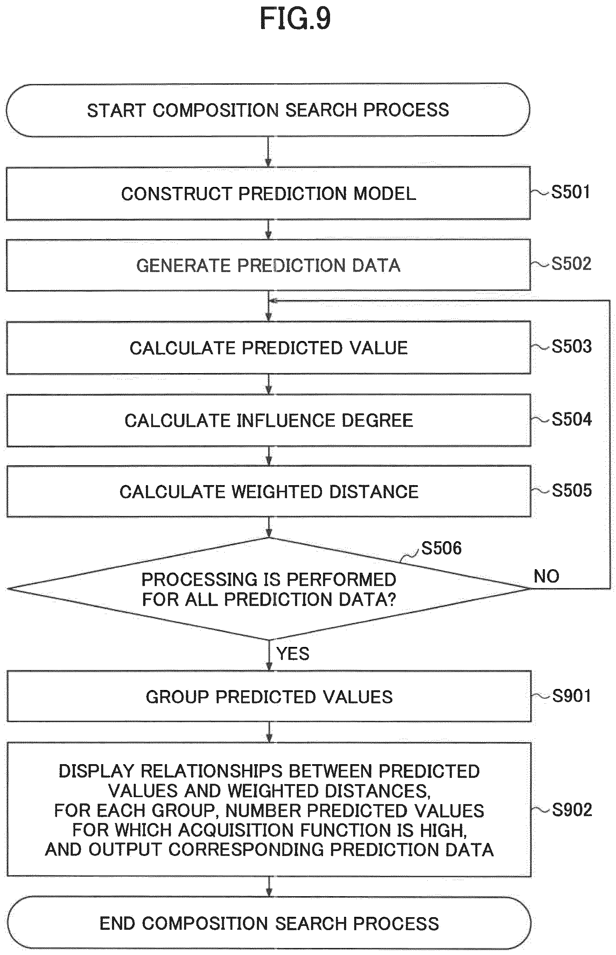

Next, the flow of the composition search process in the composition search system 100 will be described. FIG. 9 is a second flowchart illustrating the flow of the composition search process.

Here, in FIG. 9, the processing from step S501 to step S506 is substantially the same as the processing described in the first embodiment with reference to FIG. 5, and the description thereof will be omitted here.

In subsequent step S901, the predicting device 120 groups the prediction values by the weighted distances.

In step S902, the predicting device 120 displays the relationships between the predicted values and the weighted distances, and outputs the corresponding prediction data as the search candidate in the order in which the acquisition function is higher for each group. When displaying the relationships between the prediction values and the weighted distances, as illustrated in FIG. 7, the predicting device 120 plots the prediction values on a two dimensional graph with the weighted distance on the horizontal axis and the prediction value on the vertical axis, and then numbers and displays the prediction values for which the acquisition function is high, and outputs the corresponding prediction data as the search candidates.

<Conclusion>

As is clear from the above description, in the composition search method according to the second embodiment, the predicted values are grouped by the weighted distances, and the relationships between the predicted values and the weighted distances are displayed. With this, according to the composition search method of the second embodiment, the prediction data for a high predicted value can be selected in the level of the challenge property for each group and the prediction data can be output as the search candidates.

Additionally, in the composition search method according to the second embodiment, the acquisition function of the prediction data is calculated, and the prediction data corresponding to the predicted value for which the calculated acquisition function is high is output as the search candidate. With this, according to the composition search method of the second embodiment, the search candidate can be output while balancing the level of the reliability and the level of the challenge property of the predicted value.

[Third Embodiment]

Subsequently, a composition search method according to a third embodiment will be described focusing on differences from the first and second embodiments described above.

<System Configuration of Composition Search System>

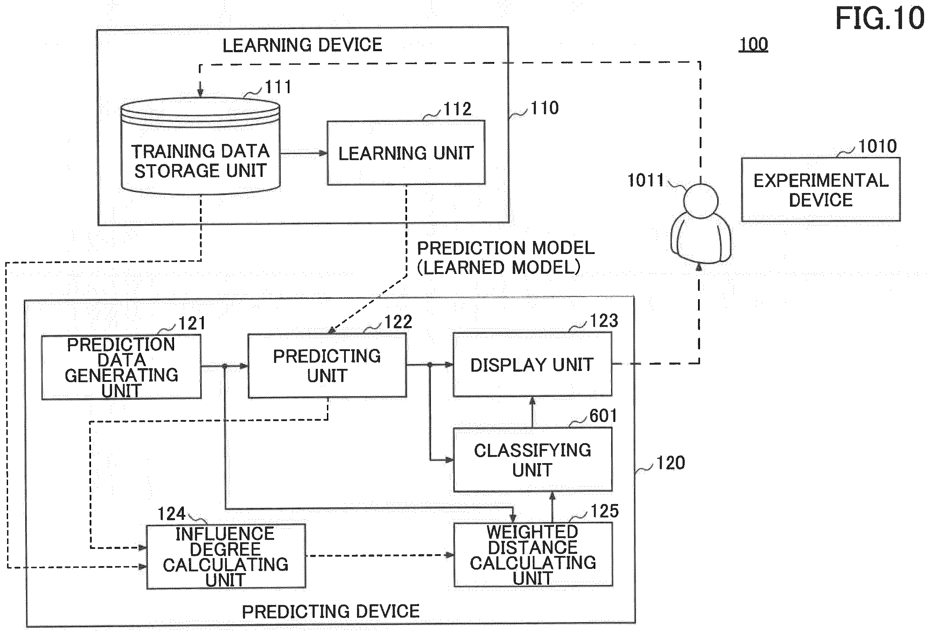

First, a system configuration of a composition search system for realizing the composition search method according to the third embodiment will be described using FIG. 10. FIG. 8 is a third diagram illustrating an example of the system configuration of the composition search system.

A difference from the system configuration described using FIG. 6 in the second embodiment is that the system configuration illustrated in FIG. 10 includes an experimental device 1010.

The experimental device 1010 is a device used when an experimenter 1011 evaluates the physical property with respect to a composition of the output search candidate. The experimenter 1011 confirms whether the value of the physical property obtained by evaluating the physical property by using the experimental device 1010 reaches the target value, and ends the search for the composition if the target value is reached. If the target value is not reached, the experimenter 1011 adds, to the training data, a set of information related to the composition of the search candidate on which the experiment has been performed and the obtained value of the physical property, and stores the training data in the training data storage unit 111.

<Flow of Composition Search Process in Composition Search System>

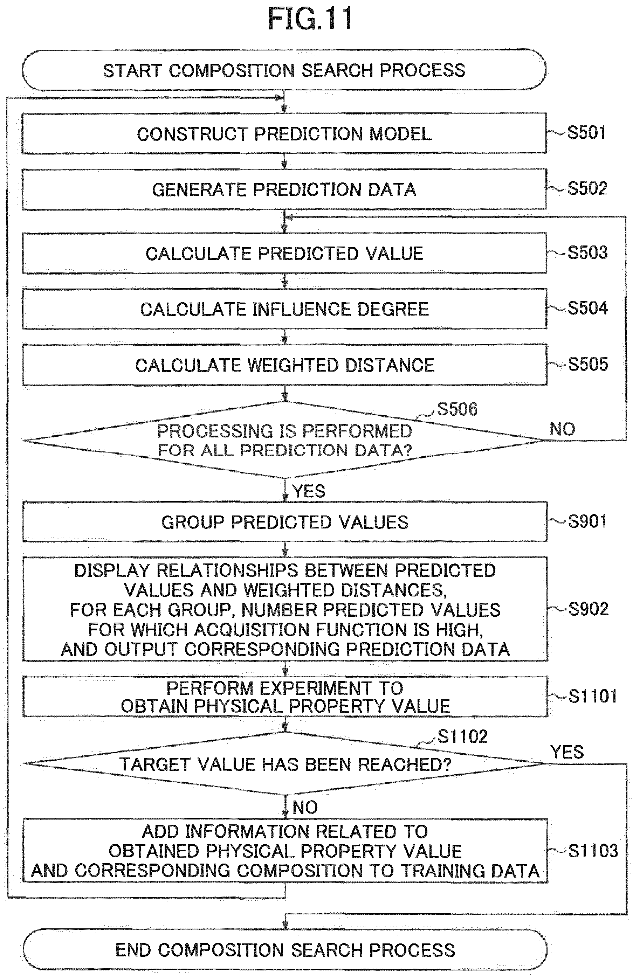

Next, the flow of the composition search process in the composition search system 100 will be described. FIG. 11 is a third flowchart illustrating the flow of the composition search process.

Here, in FIG. 11, the processing from step S501 to step S902 is substantially the same as the processing described using FIG. 9 in the second embodiment, and the description thereof will be omitted here.

In subsequent step S1101, the experimenter 1011 uses the experimental device 1010 to evaluate the physical property with respect to the composition of the search candidate output in step S902 by using the experimental device 1010, and obtains the value of the physical property.

In step S1102, the experimenter 1011 confirms whether the value of the physical property obtained in step S1101 reaches the target value. If the target value is reached (YES in step S1102), the search for the composition is ended. If the target value is not reached (NO in step S1102), the process proceeds to step S1103.

In step S1103, the experimenter 1011 adds, to the training data, the set of the information related to the composition of the search candidate on which the experiment has been performed in step S1101 and the obtained value of the physical property, and then returns to step S501. In the composition search system 100, respective steps of step S501 to step S1103 described above are repeated until the value of the physical property reaches the target value in the step S1102 by using the updated training data.

<Conclusion>

As is clear from the above description, in the composition search method according to the third embodiment, the physical property is evaluated with respect to the composition of the search candidate, and when the value of the physical property does not reach the target value, the set of the information related to the composition of the search candidate and the obtained value of the physical property is added to the training data.

As described, by using the configuration to evaluate the physical property by the experiment with respect to the search candidate in which the level of the reliability and the level of the challenge property of the predicted value are balanced, the number of experiments until the value of the physical property reaches the target value can be reduced.

Here, in the above description, the process when the value of the physical property reaches the target value is not mentioned, but when the value of the physical property reaches the target value, for example, the material is designed and produced based on the corresponding search candidate. This enables a material having the target physical property to be designed and produced.

Example

In the following, a specific example of the composition search method according to the third embodiment among the above-described embodiments will be described.

In the present example, a dataset of the paper of Turab Lookman et al. (https://www.nature.com/articles/s41598-018-21936-3#Sec12)), in which a composition of a metallic compound, a feature related to the composition, and a physical property are described, is used as the training data and the prediction data. The dataset is a modulus dataset for 223 $M_2AX$ chemical compound compositions (M: a transition metal, A: a p-block element, X: nitrogen (N) or carbon (C)), some of which are indicated in Table 1. p, d, and s orbital radii of each element in the element sites (M, A, and X) are described in the second column to the eighth column of Table 1, and these are used as the explanatory variables of the training data and the prediction data. Additionally, the Young's modulus in the ninth column is used as the objective variable of the training data.

[Table 1]

|  | M-atom p-orbital radii | M-atom d-orbital radii | M-atom s-orbital radii | A-atom s-orbital radii | A-atom p-orbital radii | X-atom s-orbital radii | X-atom p-orbital radii | Young's modulus |
|---|---|---|---|---|---|---|---|---|
| 1 | 0.5 | 0.539 | 1.57 | 0.445 | 1.184 | 0.62 | 0.596 | 92 |

(continued)

|  | M-atom p-orbital radii | M-atom d-orbital radii | M-atom s-orbital radii | A-atom s-orbital radii | A-atom p-orbital radii | X-atom s-orbital radii | X-atom p-orbital radii | Young's modulus |
|---|---|---|---|---|---|---|---|---|
| 2 | 0.5 | 0.539 | 1.57 | 1.06 | 1.319 | 0.62 | 0.596 | 135 |
| 3 | 0.5 | 0.539 | 1.57 | 1.093 | 1.382 | 0.62 | 0.596 | 135 |
| 4 | 0.5 | 0.539 | 1.57 | 1.01 | 1.215 | 0.62 | 0.596 | 142 |
| 5 | 0.5 | 0.539 | 1.57 | 1.044 | 1.312 | 0.62 | 0.596 | 140 |
| 6 | 0.5 | 0.539 | 1.57 | 0.96 | 1.254 | 0.62 | 0.596 | 133 |
| 7 | 0.5 | 0.539 | 1.57 | 0.445 | 1.184 | 0.521 | 0.4875 | 106 |
| 8 | 0.5 | 0.539 | 1.57 | 1.027 | 1.24 | 0.62 | 0.596 | 154 |
| 9 | 0.5 | 0.539 | 1.57 | 1.01 | 1.215 | 0.521 | 0.4875 | 165 |
|  | ... | ... | ... | ... | ... | ... | ... | ... |
| 223 | 0.599 | 0.784 | 1.413 | 0.803 | 0.9175 | 0.521 | 0.4875 | 315 |

The search for the optimum composition by repeating the output (the selection and proposal) of the search candidate and the evaluation (the measurement) of the physical property by the experiment was reproduced by Example 1 and Comparative Examples 1 and 2 by using the dataset described above. Specifically, the numbers of times until a composition in which the Young's modulus is the highest is found in the dataset are compared. It can be said that as the number of times becomes smaller, the method can search for the optimum composition more efficiently.

Example 1 indicates a case of performing the composition search according to the flowchart of FIG. 11, which is the composition search method according to the third embodiment. In order to compare the effects of weighting, Comparative Example 1 indicates a case of performing the composition search without performing the processing of steps S504 and S505 in the flowchart of FIG. 11. Additionally, Comparative Example 2 indicates a case of performing the composition search by a composition search method of simply outputting corresponding prediction data as the search candidates in the order in which the predicted value is high, without considering the distance from the training data.

In the following, the procedure of Example 1 will be described specifically.

In steps S501 and S502, the learning device 110 extracts, as the training data to be used first, a combination of the orbital radii and the Young's modulus of each of 24 elements having low Young's modulus among the 223 chemical compound compositions included in the dataset. Additionally, the learning device 110 sets the remaining 199 chemical compound compositions included in the dataset as the explanatory variables (the orbital radius of the respective elements) of the prediction data. The learning device 110 then performs learning using a random forest regression model of scikit-learn as a technique of the prediction model to construct the prediction model.

In step S503, the predicting device 120 calculates the predicted value from the prediction data by using the prediction model constructed in step S501.

In step S504, the predicting device 120 calculates Gini importance included in scikit-learn as the influence degree.

In step S505, the predicting device 120 calculates the weighted distances by using the influence degree calculated in step S504. The predicting device 120 repeats the steps S503 to S506 to calculate the predicted values and the weighted distances for all the prediction data, and then proceeds to step S901.

In step S901, the predicting device 120 groups the prediction data according to the weighted distances. Here, the weighted distances are divided into three groups by a method of dividing the weighted distances by a predetermined numerical value.

In step S902, the predicting device 120 outputs one composition from each group as the search candidate. Specifically, the predicting device 120 uses the above-described Equation (4) as the acquisition function, sets $s_g$ to 0 in all groups, and outputs the corresponding prediction data as the search candidate in the order in which the acquisition function is high in each group.

In step S1101, the experimenter 1011 acquires Young's modulus corresponding to the output search candidate (= the prediction data) from the dataset, instead of performing the experiment and measurement on the output search candidate.

In step S1102, the experimenter 1011 confirms whether the Young's modulus acquired in step S1101 reaches the target value (the highest value in the dataset). If the Young's modulus reaches the target value, the search is ended, and the number of times for ending of the search is obtained. If the Young's modulus does not reach the target value, the process proceeds to the next step S1103.

In step S1103, the experimenter 1011 adds a set of the information related to the output composition of the search candidate and the obtained value of the physical property to the training data for updating, and returns to step S501 of constructing the prediction model. The experimenter 1011 has repeated the above steps until the Young modulus reaches the target value in step S1102. That is, by adopting one search candidate from each group, the prediction data is reduced by three as a whole of the three groups, and the orbital radius and the corresponding Young's modulus of each element, which are the prediction data, are added to the training data.

Here, the random forest regression model used in Example 1 has randomness in search, and it is conceivable that a search candidate having the highest Young's modulus may be found for the first time by chance. Therefore, in order to appropriately compare the numbers of times until the search ends, in Example 1, Comparative Example 1, and Comparative Example 2, the procedure until the target value is reached in the step S1102 described above is repeated 100 times to acquire 100 numbers of times for ending of the search, and average values and standard deviations thereof are calculated and compared.

A difference between the procedure of Comparative Example 1 and that of Example 1 will be described specifically.

In Comparative Example 1, the processing corresponding to step S503 in Example 1 is not performed, and distances that are not weighted are calculated by setting all the influence degrees $w_t$ of the explanatory variables in the above Equation (2) to 1 in step S504. Additionally, in step S901, the distances that are not weighted are used instead of the weighted distances. The other procedures are performed as in Example 1.

A difference between the procedure of Comparative Example 2 and that of Example 1 will be described specifically.

In Comparative Example 2, the processing corresponding to steps S503, S504, S901, and S1101 in Example 1 is not performed, and three corresponding prediction data are output as the search candidates in the order in which the predicted value obtained in step S502 is higher, and then step S1101 is performed. The other procedures performed as in Example 1.

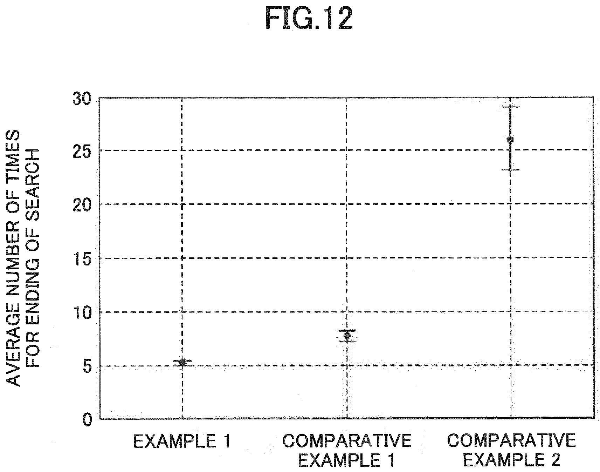

Results are indicated in Table 2 and FIG. 12. The average number of times for ending of the search was 5.2 times in Example 1, 7.7 times in Comparative Example 1, and 26.0 times in Comparative Example 2, and Example 1 indicates the smallest number of times. Table 2 indicates the average value and standard deviation of the numbers of times for ending of the search. The average numbers of times for ending of the search in Example 1 and Comparative Examples 1 and 2 are plotted in FIG. 12, and the standard deviations are indicated as error bars.

Because a value of the average number of times for ending of the search in Comparative Example 2 is clearly large, it can be said that Comparative Example 2 is less efficient than Example 1 and Comparative Example 1. A difference between the results of Example 1 and Comparative Example 1 was tested by the null hypothesis, which is a hypothesis that the effect does not exist if there is no difference between two groups. The null hypothesis is that there is no difference in the average value between the two groups. As a specific statistical method, Student's t-test was performed. As a result of the test, the p value was less than or equal to 0.01, which is the significance level, and the null hypothesis was rejected. It was determined that there was a significant difference in the number of times for ending of the search between Example 1 and Comparative Example 1 at the significance level 1%. This confirms that the composition search method according to the third embodiment is a method that can efficiently search for the composition.

[Table 2]

|  | AVERAGE NUMBER OF TIMES FOR ENDING OF SEARCH (STANDARD DEVIATION) |
|---|---|
| EXAMPLE 1 | 5.2 ($\pm$1.4) |
| COMPARATIVE EXAMPLE 1 | 7.7 ($\pm$2.4) |
| COMPARATIVE EXAMPLE 2 | 26.0 ($\pm$14.5) |

Industrial availability

The composition search method of the present invention can be used for the material design in alloy materials, organic materials, composite materials, and the like.

**Claims**

1. A composition search method for a material, comprising:

a step of constructing a prediction model by learning training data in which information related to a composition of a material is set as an explanatory variable and a value of a physical property of the material is set as an objective variable;

a step of calculating a predicted value of the physical property by inputting, into the prediction model, prediction data for newly searching for a composition;

a step of calculating an influence degree of each explanatory variable on prediction by using the training data and the prediction model;

a step of calculating a weighted distance of the prediction data with respect to the training data by using the influence degree; and

a step of displaying a relationship between the predicted value and the weighted distance, and outputting corresponding prediction data as a search candidate.

2. The composition search method as claimed in claim 1, wherein in the step of calculating the weighted distance, the weighted distance is scaled to a value between zero and one, inclusive.

3. The composition search method as claimed in claim 1,

wherein the prediction data are a combination of information related to the composition exhaustively generated according to a constraint condition of a step size or a composition ratio that is set in advance, and

wherein by repeating the step of calculating the predicted value of the physical property to the step of calculating the weighted distance, in the step of displaying the relationship between the predicted value and the weighted distance, a plurality of said relationships between the said calculated predicted values and the said weighted distances are displayed.

4. The composition search method as claimed in claim 3, further comprising a step of grouping the predicted values by the weighted distances, and

wherein in the step of displaying the relationship between the predicted value and the weighted distance, the prediction data are divided into groups and output.

5. The composition search method as claimed in claim 4, wherein in the step of displaying the relationship between the predicted value and the weighted distance, corresponding prediction data are output as search candidates in an order in which the predicted value is higher for each of the groups.

6. The composition search method as claimed in claim 4, wherein in the step of grouping, the grouping is performed by equally dividing the weighted distances by a predetermined value between zero and one.

7. The composition search method as claimed in claim 4, wherein in the step of grouping, the grouping is performed by dividing the weighted distance between zero and one such that a number of the predicted values in a group after the division is identical.

8. The composition search method as claimed in claim 3, wherein in the step of displaying the relationship between the predicted value and the weighted distance, a number of the prediction data to be output as the search candidate is set by a user.

9. The composition search method as claimed in claim 4, further comprising:

a step of calculating an acquisition function $Acq(X_i)$ with respect to the predicted value and the weighted distance calculated from the prediction data by using the following Equation (1); and

a step of outputting corresponding prediction data as the search candidates in an order in which the calculated acquisition function is higher, [Eq. 1]

$$Acq(X_i) = (1 - s_g) * f(X_i) + s_g * D_i \qquad (0 \leq s_g \leq 1) \qquad (1)$$

where $X_i$ is the i-th prediction data, $f(X_i)$ is a predicted value of $X_i$ scaled to a value between zero and one, inclusive, $s_g$ is a weighting factor in the g-th group, and $D_i$ is the weighted distance of $X_i$.

10. The composition search method as claimed in claim 3, further comprising:

a step of performing an experiment based on the information related to the composition of the prediction data output as the search candidate in the step of outputting, to obtain a value of the physical property; and

a step of adding the information related to the composition corresponding to the obtained value of the physical property to the training data,

wherein processing of constructing the prediction model by using the training data to which data is added in the step of constructing the prediction model to processing of obtaining the value of the physical property in the step of obtaining the value of the physical property are repeated until the obtained value of the physical property reaches a predetermined target value.

**Patentansprüche**

1. Zusammensetzungssuchverfahren für ein Material, mit:

einem Schritt des Konstruierens eines Vorhersagemodells durch Lernen von Trainingsdaten, wobei Information in Bezug auf eine Zusammensetzung eines Materials als erklärende Variable gesetzt wird und ein Wert einer physikalischen Eigenschaft des Materials als Zielvariable gesetzt wird;

einem Schritt des Berechnens eines vorhergesagten Werts der physikalischen Eigenschaft durch Eingeben von Vorhersagedaten zum neuen Suchen nach einer Zusammensetzung in das Vorhersagemodell;

einem Schritt des Berechnens eines Einflussgrads jeder erklärenden Variablen auf die Vorhersage unter Verwendung der Trainingsdaten und des Vorhersagemodells;

einem Schritt des Berechnens eines gewichteten Abstands der Vorhersagedaten in Bezug auf die Trainingsdaten unter Verwendung des Einflussgrads; und

einem Schritt des Anzeigens einer Beziehung zwischen dem vorhergesagten Wert und dem gewichteten Abstand und des Ausgebens entsprechender Vorhersagedaten als Suchkandidat.

2. Zusammensetzungssuchverfahren nach Anspruch 1, wobei in dem Schritt des Berechnens des gewichteten Abstands der gewichtete Abstand auf einen Wert zwischen null und einschließlich eins skaliert wird.

3. Zusammensetzungssuchverfahren nach Anspruch 1,

wobei die Vorhersagedaten eine Kombination von Informationen betreffend die Zusammensetzung sind, die erschöpfend entsprechend einer Nebenbedingung einer Schrittgröße oder eines Zusammensetzungsverhältnisses, das im Voraus gesetzt wird, erzeugt werden, und

wobei, durch Wiederholen des Schritts des Berechnens des vorhergesagten Werts der physikalischen Eigenschaft bis zu dem Schritt des Berechnens des gewichteten Abstands, in dem Schritt des Anzeigens der Beziehung zwischen dem vorhergesagten Wert und dem gewichteten Abstand eine Vielzahl der Beziehungen zwischen den berechneten vorhergesagten Werten und den gewichteten Abständen angezeigt werden.

4. Zusammensetzungssuchverfahren nach Anspruch 3, welches weiterhin einen Schritt des Gruppierens der vorhersagten Werte mittels der gewichteten Abstände aufweist, und

wobei in dem Schritt des Anzeigens der Beziehung zwischen dem vorhergesagten Wert und dem gewichteten Abstand die Vorhersagedaten in Gruppen unterteilt werden und ausgegeben werden.

5. Zusammensetzungssuchverfahren nach Anspruch 4, wobei, in dem Schritt des Anzeigens der Beziehung zwischen dem vorhergesagten Wert und dem gewichteten Abstand, entsprechende Vorhersagedaten als Suchkandidaten in einer Reihenfolge ausgegeben werden, in der der vorhergesagte Wert für jede der Gruppen höher ist.

6. Zusammensetzungssuchverfahren nach Anspruch 4, wobei in dem Schritt des Gruppierens das Gruppieren bewerkstelligt wird, indem die gewichteten Abstände in einen vorbestimmten Wert zwischen null und eins in gleiche Teile eingeteilt werden.

7. Zusammensetzungssuchverfahren nach Anspruch 4, wobei in dem Schritt des Gruppierens das Gruppieren durch Einteilen des gewichteten Abstands zwischen null und eins derart durchgeführt wird, dass eine Anzahl der vorhergesagten Werte in einer Gruppe nach dem Einteilen identisch ist.

8. Zusammensetzungssuchverfahren nach Anspruch 3, wobei in dem Schritt des Anzeigens der Beziehung zwischen dem vorhergesagten Wert und dem gewichteten Abstand eine Anzahl der Vorhersagedaten, die als Suchkandidat ausgegeben werden sollen, durch einen Benutzer gesetzt wird.

9. Zusammensetzungssuchverfahren nach Anspruch 4, weiterhin umfassend:

einen Schritt des Berechnens einer Akquisitionsfunktion Acq($X_i$) in Bezug auf den vorhergesagten Wert und den gewichteten Abstand, die aus den Vorhersagedaten unter Verwendung der folgenden Gleichung (1) berechnet wird; und
einen Schritt des Ausgebens entsprechender Vorhersagedaten als Suchkandidaten in einer Reihenfolge, in der die berechnete Akquisitionsfunktion höher ist,

[Gl. 1]

$$Acq(X_i) = (1-s_g) * f(X_i) + sg * D_i \qquad (0 \leq s_g \leq 1)$$

wobei $X_i$ die i-ten Vorhersagedaten sind, $f(X_i)$ ein vorhergesagter Wert von $X_i$ ist, der auf einen Wert zwischen null und einschließlich eins skaliert ist, $s_g$ ein Gewichtungsfaktor in der g-ten Gruppe ist und $D_i$ der gewichtete Abstand von $X_i$ ist.

10. Zusammensetzungssuchverfahren nach Anspruch 3, weiterhin umfassend:

einen Schritt des Durchführens eines Experiments basierend auf den Informationen betreffend die Zusammensetzung der Vorhersagedaten, die als Suchkandidat in dem Schritt des Ausgebens ausgegeben werden, um einen Wert der physikalischen Eigenschaft zu erhalten; und
einen Schritt des Hinzufügens der Informationen betreffend die Zusammensetzung, entsprechend dem erhaltenen Wert der physikalischen Eigenschaft, zu den Trainingsdaten,
wobei eine Verarbeitung des Konstruierens des Vorhersagemodells unter Verwendung der Trainingsdaten, zu denen Daten in dem Schritt des Konstruierens des Vorhersagemodells hinzugefügt werden, bis zu einer Verarbeitung des Erhaltens des Werts der physikalischen Eigenschaft in dem Schritt des Erhaltens des Werts der physikalischen Eigenschaft wiederholt wird, bis der erhaltene Wert der physikalischen Eigenschaft einen vorbestimmten Zielwert erreicht.

**Revendications**

1. Procédé de recherche de composition pour un matériau, comprenant :

une étape de construction d'un modèle de prédiction par apprentissage de données d'entraînement dans laquelle les informations relatives à la composition d'un matériau sont définies comme variable explicative et une valeur d'une propriété physique du matériau comme variable objective ;
une étape consistant à calculer une valeur prédite de la propriété physique en saisissant, dans le modèle de prédiction, des données de prédiction pour la recherche d'une nouvelle composition ;
une étape consistant à calculer le degré d'influence de chaque variable explicative sur la prédiction en utilisant les données d'entraînement et le modèle de prédiction ;
une étape consistant à calculer une distance pondérée entre les données de prédiction et les données d'entraînement en utilisant le degré d'influence ; et
une étape consistant à afficher une relation entre la valeur prédite et la distance pondérée, et à produire les données de prédiction correspondantes en tant que candidat à la recherche.

2. Procédé de recherche de composition selon la revendication 1, dans lequel, à l'étape de calcul de la distance pondérée, la distance pondérée est mise à l'échelle à une valeur comprise entre zéro et un inclus.

3. Procédé de recherche de composition selon la revendication 1,
dans lequel les données de prédiction sont une combinaison d'informations relatives à la composition, générées de manière exhaustive selon une condition de contrainte de taille de pas ou de rapport de composition défini à l'avance, et dans lequel, en répétant l'étape de calcul de la valeur prédite de la propriété physique jusqu'à l'étape de calcul de la distance pondérée, à l'étape d'affichage de la relation entre la valeur prédite et la distance pondérée, une pluralité desdites relations entre lesdites valeurs prédites calculées et lesdites distances pondérées sont affichées.

4. Procédé de recherche de composition selon la revendication 3, comprenant en outre une étape de regroupement des valeurs prédites par les distances pondérées, et
dans lequel, lors de l'étape d'affichage de la relation entre la valeur prédite et la distance pondérée, les données de prédiction sont divisées en groupes et affichées.

5. Procédé de recherche de composition selon la revendication 4, dans lequel, à l'étape d'affichage de la relation entre la valeur prédite et la distance pondérée, les données de prédiction correspondantes sont affichées comme candidats à la recherche dans un ordre où la valeur prédite est plus élevée pour chacun des groupes.

6. Procédé de recherche de composition selon la revendication 4, dans lequel, à l'étape de regroupement, le regroupement est effectué en divisant de manière égale les distances pondérées par une valeur prédéterminée comprise entre zéro et un.

7. Procédé de recherche de composition selon la revendication 4, dans lequel, à l'étape de regroupement, le regroupement est effectué en divisant la distance pondérée entre zéro et un de sorte qu'un certain nombre de valeurs prédites dans un groupe après la division soient identiques.

8. Procédé de recherche de composition selon la revendication 3, dans lequel, à l'étape d'affichage de la relation entre la valeur prédite et la distance pondérée, un certain nombre de données de prédiction à afficher comme candidat de recherche est défini par un utilisateur.

9. Procédé de recherche de composition selon la revendication 4, comprenant en outre :

une étape de calcul d'une fonction d'acquisition Acq(Xi) par rapport à la valeur prédite et à la distance pondérée calculée à partir des données de prédiction en utilisant l'équation suivante (1) ; et
une étape consistant à produire les données de prédiction correspondantes comme candidats de recherche dans un ordre où la fonction d'acquisition calculée est plus élevée,
[Éq. 1]

$$Acq(X_i) = (1 - s_g) * f(X_i) + s_g * D_i \qquad (0 \le s_g \le 1) \qquad (1)$$

où $X_i$ représente la i-ème donnée de prédiction, $f(X_i)$ est une valeur prédite de $X_i$ mise à l'échelle à une valeur comprise entre zéro et un inclus, $s_g$ est un facteur de pondération dans le g-ème groupe, et $D_i$ est la distance pondérée de $X_i$.

10. Procédé de recherche de composition selon la revendication 3, comprenant en outre :

une étape consistant à réaliser une expérience basée sur les informations relatives à la composition des données de prédiction comme candidat de recherche issues de l'étape de sortie, afin d'obtenir une valeur de la propriété physique ; et
une étape consistant à ajouter aux données d'apprentissage les informations relatives à la composition correspondant à la valeur obtenue de la propriété physique,
dans lequel le traitement de construction du modèle de prédiction, à l'aide des données d'entraînement auxquelles des données sont ajoutées à l'étape de construction du modèle de prédiction, jusqu'au traitement d'obtention de la valeur de la propriété physique à l'étape d'obtention de la valeur de la propriété physique sont répétés jusqu'à ce que la valeur obtenue de la propriété physique atteigne une valeur cible prédéterminée.

# FIG.1

100

**LEARNING DEVICE** 110

TRAINING DATA STORAGE UNIT 111

LEARNING UNIT 112

PREDICTION MODEL (LEARNED MODEL)

**PREDICTING DEVICE** 120

PREDICTION DATA GENERATING UNIT 121

PREDICTING UNIT 122

DISPLAY UNIT 123

INFLUENCE DEGREE CALCULATING UNIT 124

WEIGHTED DISTANCE CALCULATING UNIT 125

# FIG.2

110, 120

LEARNING DEVICE AND PREDICTING DEVICE

| 201 | 202 | 203 |
|---|---|---|
| PROCESSOR | MEMORY | AUXILIARY STORAGE DEVICE |

207

| 204 | 205 | 206 |
|---|---|---|
| I/F DEVICE | COMMUNICATION DEVICE | DRIVE DEVICE |

211 OPERATION DEVICE

212 DISPLAY DEVICE

213 RECORDING MEDIUM

# FIG.3

(A)

| | MAIN RAW MATERIAL A | MAIN RAW MATERIAL B | MAIN RAW MATERIAL C | ... | ADDITIVE A | ADDITIVE B | ... | PHYSICAL PROPERTY VALUE |
|---|---|---|---|---|---|---|---|---|
| SAMPLE 1 | 70 | 0 | 20 | ... | 10 | 5 | ... | 2.0 |
| SAMPLE 2 | 75 | 10 | 0 | ... | 0 | 5 | ... | 1.4 |
| ... | | | | | | | | |
| SAMPLE N | 70 | 15 | 0 | ... | 5 | 0 | ... | 3.2 |

⎫ N LEARNING DATA

(B)

| | MAIN RAW MATERIAL A | MAIN RAW MATERIAL B | MAIN RAW MATERIAL C | ... | ADDITIVE A | ADDITIVE B | ... |
|---|---|---|---|---|---|---|---|
| SAMPLE 1 | 60 | 0 | 20 | ... | 10 | 0 | ... |
| SAMPLE 2 | 60 | 5 | 15 | ... | 8 | 2 | ... |
| SAMPLE 3 | 65 | 10 | 10 | ... | 6 | 4 | ... |
| SAMPLE 4 | 65 | 15 | 5 | ... | 10 | 6 | ... |
| ... | | | | | | | |
| SAMPLE M | 80 | 0 | 20 | ... | 0 | 10 | ... |

⎫ M PREDICTION DATA

EP 4 414 993 B1

# FIG.4

PREDICTION DATA 1, PREDICTED VALUE 1, WEIGHTED DISTANCE 1
PREDICTION DATA 2, PREDICTED VALUE 2, WEIGHTED DISTANCE 2
PREDICTION DATA 3, PREDICTED VALUE 3, WEIGHTED DISTANCE 3
.
.
.

# FIG.5

START COMPOSITION SEARCH PROCESS

CONSTRUCT PREDICTION MODEL — S501

GENERATE PREDICTION DATA — S502

CALCULATE PREDICTED VALUE — S503

CALCULATE INFLUENCE DEGREE — S504

CALCULATE WEIGHTED DISTANCE — S505

S506
PROCESSING IS PERFORMED
FOR ALL PREDICTION DATA? — NO

YES

DISPLAY RELATIONSHIPS BETWEEN PREDICTED
VALUES AND WEIGHTED DISTANCES AND
OUTPUT CORRESPONDING PREDICTION DATA
AS SEARCH CANDIDATE — S507

END COMPOSITION SEARCH PROCESS

FIG.6

EP 4 414 993 B1

# FIG.7

GROUP 1
① PREDICTION DATA 1, PREDICTED VALUE 1, WEIGHTED DISTANCE 1
GROUP 2
② PREDICTION DATA 23, PREDICTED VALUE 23, WEIGHTED DISTANCE 23
GROUP 3
③ PREDICTION DATA 67, PREDICTED VALUE 67, WEIGHTED DISTANCE 67

# FIG.8

① PREDICTION DATA 1, PREDICTED VALUE 1, WEIGHTED DISTANCE 1
② PREDICTION DATA 4, PREDICTED VALUE 4, WEIGHTED DISTANCE 4
③ PREDICTION DATA 23, PREDICTED VALUE 23, WEIGHTED DISTANCE 23

# FIG.9

START COMPOSITION SEARCH PROCESS

CONSTRUCT PREDICTION MODEL — S501

GENERATE PREDICTION DATA — S502

CALCULATE PREDICTED VALUE — S503

CALCULATE INFLUENCE DEGREE — S504

CALCULATE WEIGHTED DISTANCE — S505

S506
PROCESSING IS PERFORMED
FOR ALL PREDICTION DATA?　　NO

YES

GROUP PREDICTED VALUES — S901

DISPLAY RELATIONSHIPS BETWEEN PREDICTED
VALUES AND WEIGHTED DISTANCES,
FOR EACH GROUP, NUMBER PREDICTED VALUES — S902
FOR WHICH ACQUISITION FUNCTION IS HIGH,
AND OUTPUT CORRESPONDING PREDICTION DATA

END COMPOSITION SEARCH PROCESS

FIG.10

# FIG.11

START COMPOSITION SEARCH PROCESS

CONSTRUCT PREDICTION MODEL — S501

GENERATE PREDICTION DATA — S502

CALCULATE PREDICTED VALUE — S503

CALCULATE INFLUENCE DEGREE — S504

CALCULATE WEIGHTED DISTANCE — S505

S506
PROCESSING IS PERFORMED
FOR ALL PREDICTION DATA? — NO

YES

GROUP PREDICTED VALUES — S901

DISPLAY RELATIONSHIPS BETWEEN PREDICTED
VALUES AND WEIGHTED DISTANCES,
FOR EACH GROUP, NUMBER PREDICTED VALUES — S902
FOR WHICH ACQUISITION FUNCTION IS HIGH,
AND OUTPUT CORRESPONDING PREDICTION DATA

PERFORM EXPERIMENT TO
OBTAIN PHYSICAL PROPERTY VALUE — S1101

S1102
TARGET VALUE HAS BEEN REACHED? — YES

NO

ADD INFORMATION RELATED TO
OBTAINED PHYSICAL PROPERTY VALUE — S1103
AND CORRESPONDING COMPOSITION TO TRAINING DATA

END COMPOSITION SEARCH PROCESS

# FIG.12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020187642 A

- WO 2020152993 A

**Non-patent literature cited in the description**

- Materials Informatics Approach to Predictive Models for Elastic Modulus of Polypropylene Composites Reinforced by Fillers and Additives. **IKEDA YUKO et al.** JOURNAL OF COMPUTER CHEMISTRY, JAPAN - INTERNATIONAL EDITION. 11 June 2021, vol. 7, 1-8